# EUROPÄISCHE PATENTSCHRIFT

(11) **EP 2 582 416 B1**
(45) Veröffentlichungstag und Bekanntmachung des Hinweises auf die Patenterteilung: **02.09.2020**
(21) Anmeldenummer: 11726140.4
(22) Anmeldetag: 15.06.2011
(51) Int. Cl.: A61M 5/31, A61M 5/00

(54) **HALTERUNG FÜR EINE INJEKTIONSSPRITZE**
HOLDER FOR AN INJECTION SYRINGE
SUPPORT POUR UNE SERINGUE D'INJECTION

(30) Priorität: 15.06.2010 EP 10165924
(43) Veröffentlichungstag der Anmeldung: 24.04.2013
(73) Patentinhaber: CSL Behring GmbH, 35041 Marburg (DE)
(72) Erfinder: PLÜNNECKE, Dieter, 34233 Fuldatal (DE)
(74) Vertreter: Trösch, Dominique
(86) Internationale Anmeldenummer: PCT/EP2011/059904
(87) Internationale Veröffentlichungsnummer: WO 2011/157737

(56) Entgegenhaltungen:
- EP-A1- 0 790 063
- EP-A1- 1 486 219
- WO-A1-2005/102416
- WO-A1-2007/012915
- WO-A1-2010/054290
- DE-C- 352 141
- DE-U1-202005 014 437
- US-A- 2 455 757

## Beschreibung

Die vorliegende Erfindung betrifft eine Halterung für eine Spritze zu Selbstmedikationszwecken, insbesondere zum Abstellen auf einer Unterlage.

Aus EP 0 790 063 A1 sind für Verpackungs- und Handhabungsvorrichtungen vorgesehene und ausgebildete Halterungen für eine Vielzahl von Spritzenzylindern zum Abstellen auf Unterlagen bekannt. Aus DE 20 2005 014 437 U1 ist ein Spritzenstand zur automatischen Perfusion von Explantaten bekannt, der auf einer Unterlage abgestellt werden kann. Bekannt aus WO 2005/102416, WO 2007/012915 A1, EP 486 219 A1 sind außerdem Vorrichtungen zur Aufnahme von Spritzen in Injektor- oder Perfusionsvorrichtungen, bei denen der Spritzenstößel maschinell bewegt wird. Aus WO 2010/054290 A1 ist ferner eine Spritzenaufnahme zum Aufhängen bekannt, und aus der deutschen Reichspatentschrift Nr. 352141 ist eine in einem Alkoholbehälter aufbewahrte Injektionsspritze bekannt.

Insbesondere im Rahmen einer intravenösen oder subkutanen Darreichungsform von Medikamenten wird der zumeist flüssige Wirkstoff mit Hilfe einer Spritze oder vergleichbaren Injektionsgeräten dem Körpergewebe zugeführt. Insbesondere bei der Selbstmedikation oder Heimtherapie erfordern derartige Medikamente eine entsprechende Schulung des Patienten. Soll beispielsweise ein Hämophilie-Präparat verabreicht werden, so geschieht dies zumeist unter Verwendung einer sogenannten Butterfly-Kanüle.

Diese legt sich ein Patient selbst z.B. im Bereich der Armbeuge. Für den eigentlichen Injektionsvorgang steht dem Patienten dann jedoch nur noch eine Hand zur Verfügung. Hinzu kommt, dass das zu verabreichende Präparat eine im Vergleich zu Wasser höhere Viskosität aufweist, so dass der Patient zur Injektion des Wirkstoffs eine erhebliche Kraft auf einen Spritzenkolben ausüben muss. Eine derartige einhändige Handhabung der Injektionsspritze ist vergleichsweise umständlich und beschwerlich. Dabei besteht insbesondere die Gefahr, dass ein den Spritzenauslass mit einer Injektionskanüle verbindender fluidführender Schlauch beim Injektionsvorgang abgeknickt wird, was die Zuführung des Präparats weiter erschwert.

Weiter gilt es zu berücksichtigen, dass insbesondere Hämophilie-Patienten bereits an Sekundärerkrankungen leiden und hinsichtlich ihrer Beweglichkeit und Fingerfertigkeit oftmals eingeschränkt sind. Versteifte Gelenke gerade im Unterarm- und Handbereich können die Selbstmedikation erheblich erschweren.

Es ist daher Aufgabe der vorliegenden Erfindung, eine Halterung für eine Injektionsspritze bereitzustellen, die eine einfache, sichere sowie zuverlässige Injektion ermöglicht und die Handhabung einer Injektionsspritze bzw. eines Injektionssets, umfassend einen Injektionsschlauch, eine Kanüle und eine Spritze vereinfacht.

Die der Erfindung zugrundeliegende Aufgabe wird mit Hilfe einer Halterung gemäß dem unabhängigen Patentanspruch 1 gelöst. Einzelne vorteilhafte Ausgestaltungen der Erfindung sind Gegenstand der abhängigen Patentansprüche.

Die erfindungsgemäße Halterung ist insbesondere für eine Injektionsspritze ausgebildet. Sie weist ein zur Aufnahme der Spritze ausgebildetes Gehäuse und einen Standfuß zum Abstellen der Halterung auf einer Unterlage auf. Ferner sind an der Halterung Haltemittel zum axialen und/oder radialen Fixieren der Spritze an oder in der Halterung vorgesehen. Die Haltemittel sind dabei derart ausgebildet, dass ein Auslass der Spritze in der Halterung dem freien Ende des Standfußes zugewandt und beabstandet vom Standfuß, das heißt entsprechend beabstandet von der Unterlage, auf welcher die Halterung abzustellen ist, zu liegen kommt. Dabei ist insbesondere vorgesehen, dass die Haltemittel der Halterung für eine Spritzenfixierung innerhalb oder an der Halterung ausgelegt sind, sodass der Spritzenauslass bei einer auf einer Unterlage abgestellten Halterung zur Unterlage hin orientiert, aber beabstandet hiervon angeordnet ist und dass die Spritze in der Halterung vertikal, d.h. im Wesentlichen parallel zur Axialrichtung des Standfußes und der Halterung angeordnet ist.

Durch die Möglichkeit, die Halterung quasi senkrecht auf einer Unterlage abzustellen, wird bereits eine vereinfachte Handhabung der Spritze zu Injektionszwecken bereitgestellt. So kann der Anwender beispielsweise die Halterung mit vier Fingern umgreifen, während er mit dem Daumen auf den ausgezogenen Spritzenstößel eine nach unten gerichtete Injektionskraft ausübt. Hierbei erweist es sich von Vorteil, dass die Spritzenhalterung auf einer Unterlage sicher abgestellt werden kann und die zu Injektionszwecken auf den Spritzenstößel auszuübenden Kräfte über den Spritzenkörper in die Halterung und schließlich auf oder in die Unterlage abgeleitet werden können. Sollte der Anwender etwa alleine mit seinem Daumen die erforderliche Injektionskraft nicht aufbringen können, so besteht mittels der Halterung ferner die Möglichkeit, mit anderen Körperpartien, etwa mit der flachen Hand, Druck auf den Spritzenstößel auszuüben, ohne dass eine erhöhte Gefahr bestünde, den die Kanüle und die Spritze fluidführend miteinander verbindenden Schlauch abzuknicken.

Der Standfuß ist von Vorteil zum Abstellen auf einer im Wesentlichen ebenen Unterlage, etwa einem Tisch, ausgebildet. Der Fuß kann hierzu beispielsweise einen zumindest bereichsweise umlaufenden Aufstellrand aufweisen, der im Wesentlichen in der Ebene senkrecht zur Spritzenlängsrichtung liegt.

Gemäß der Erfindung ist vorgesehen, dass die für die Spritze vorgesehenen halterseitigen Haltemittel eine derartige axiale Fixierung der Spritze in oder an der Halterung ermöglichen, dass der Abstand zwischen Spritzenauslass zum Standfußende wenigstens der Axialerstreckung eines Schlauchanschlussstücks zuzüglich eines Mindest-Schlauchbiegeradius entspricht, bei welchem der mit dem Spritzenauslass in Fluidverbindung tretende Schlauch fluiddurchlässig bleibt. Auf diese Art und Weise kann erreicht werden, dass der am Spritzenauslass anzuordnende Schlauch quasi frei hängend in der die Spritze aufnehmenden Halterung liegt. Ein Abknicken des fluidführenden Schlauchs wird somit effektiv verhindert.

Vorteilhafterweise ist die Halterung geeignet unterschiedlich große Spritzen aufzunehmen, insbesondere soll die Halterung für 5-, 10- oder 20-Milliliter-Einwegspritzen ausgebildet sein. Je nach bevorzugtem Spritzentyp ist der Abstand zwischen Spritzenauslass und Standfußende an das entsprechende Schlauchanschlussstück und den entsprechenden Schlauchdurchmesser bzw. den Mindest-Schlauchbiegeradius anzupassen oder ist hieran angepasst. Der Fachmann ist hierzu aufgrund seines Fachwissens und des Wissens um die entsprechenden Spritzen- und Schlauchcharakteristika, wie z.B. Länge der Spritzen und der Anschlussstücke und Durchmesser und Flexibilität des Schlauches, in der Lage ohne selbst erfinderisch tätig werden zu müssen.

Gemäß der Erfindung ist ferner vorgesehen, dass das Gehäuse der Halterung an seinem dem Standfuß abgewandten oberen Endabschnitt einen radial nach außen ragenden, zumindest bereichsweise umlaufenden Anlageflansch aufweist. Mit diesem Anlageflansch kann ein Hindurchrutschen des Gehäuses durch eine das Gehäuse umfassende Hand des Anwenders effektiv verhindert werden. In Anwendungsszenarien, bei welchen mittels des Daumens eine Injektionskraft auf den Spritzenstößel aufzubringen ist, umschließen die übrigen vier Finger der Hand das Gehäuse der Halterung. Der radial nach außen ragende und zumindest bereichsweise umlaufende Anlageflansch kommt dabei bevorzugt an der dem Daumen zugewandten Seite eines Zeigefingers zu liegen.

Nach einer Weiterbildung der Erfindung ist ferner vorgesehen, dass sich das Gehäuse zum Standfuß hin radial erweitert. Besonders bevorzugt weist das Gehäuse eine zumindest bereichsweise zylindrische, mit einer außenseitigen Grifffläche versehene Grundstruktur auf. Die Grifffläche selbst kann beispielsweise an die Ergonomie einer menschlichen Hand angepasst sein. Sie kann beispielsweise eine dementsprechende Oberflächenstruktur, etwa eine an die einzelnen Finger einer menschlichen Hand angepasste Riffelung aufweisen. Abweichend davon kann sie natürlich auch eine im Wesentlichen glatte, bereichsweise kreisrunde oder ovale Kontur aufweisen.

Die radiale Erweiterung des Gehäuses verbessert die Standsicherheit des Gehäuses auf der Unterlage. So kann beispielsweise vorgesehen sein, dass sich das Gehäuse zum Standfuß hin konisch oder anderweitig radial erweitert. So kann selbst bei Aufwendung höchster Injektionskräfte ein unbeabsichtigtes Umfallen der Halterung oder ein Um- oder Abknicken des Spritzenschlauchs während eines Injektionsvorganges weitgehend verhindert bzw. ausgeschlossen werden.

In einer besonderen Ausführungsform werden das Gehäuse, der Standfuß und/oder der dem Standfuß gegenüberliegende Anlageflansch einstückig ausgebildet. Bevorzugt ist hier vorzusehen, das Gehäuse, den Standfuß und/oder den Anlageflansch in Form eines einzigen Spritzgussteils, bevorzugt als Kunststoff-Spritzgussbauteil auszubilden. Die Ausbildung als Kunststoff-Spritzgussbauteil ist vergleichsweise einfach und kostengünstig zu verwirklichen.

Weiterhin ist nach einer bevorzugten Ausführungsform der Erfindung vorgesehen, dass das Gehäuse eine radial zugängliche Aufnahme zum Einfügen der Spritze in das Gehäuse aufweist. Betrachtet man die Zylindersymmetrie der Spritze bzw. des das Medikament aufnehmenden Spritzenkörpers, so ist vorgesehen, die Spritze transversal zur Spritzenlängsrichtung, das heißt transversal zur Axialrichtung, in die Aufnahme des Gehäuses einzusetzen. Dementsprechend weist das Gehäuse keine strikte Zylindersymmetrie auf, sondern ist in Umfangsrichtung nur bereichsweise rund ausgebildet, während es zu der für die Spritze vorgesehenen Einführöffnung hin, beispielsweise als aufgeschnittener Hohlzylinder ausgebildet ist, der mittels eines gesonderten Verschlussmechanismus nach Einsetzen einer Spritze zum Fixieren derselben verschließbar ist.

Alternativ oder ergänzend hierzu ist ferner aber auch denkbar, die Spritze in Axialrichtung, das heißt in Spritzenlängsrichtung gesehen, in die Halterung von oben einzuführen, bis die Spritze mit ihren am oberen Ende des Spritzenkörpers nach außen auskragenden Haltevorsprüngen an dafür vorgesehenen Anschlagsflächen oder Auflagerflächen der Halterung zu liegen kommt. Die Spritze wäre dabei unter Umständen lediglich unidirektional in Axialrichtung, nämlich in Injektionsrichtung an der Halterung fixiert, während die Halterung selbst als hohlzylindrische Aufnahme für den Spritzenkörper fungiert.

Bevorzugt ist für die Erfindung vorgesehen, dass die Halterung unterschiedlich große Spritzen aufnehmen kann. Die Halterung soll insbesondere für 5-, 10- oder 20-Milliliter-Einwegspritzen ausgebildet sein und sämtliche, zumindest aber die drei genannten Spritzenformate, sowohl in Radial- als auch Axialrichtung zu Injektionszwecken fixieren können. Hierzu erweist es sich als vorteilhaft, wenn in der Spritzenaufnahme der Halterung zumindest ein Halteflügel schwenkbar gelagert ist, der mit dem Spritzenkörper mit Einsetzen der Spritze in die Aufnahme klemmend zur Anlage bringbar ist, d.h. mit welchem die Spritze klemmend in die Halterung bringbar ist. Der zumindest eine Halteflügel stellt eine Art radialen Rastmechanismus für die Spritze zur Verfügung und ermöglicht, dass sämtliche der vorgenannten Spritzengrößen mit jeweils unterschiedlichen Durchmessern in der Spritzenaufnahme der Halterung fixiert werden können.

Von Vorteil ist dabei vorgesehen, dass der zumindest eine Halteflügel an einer im Wesentlichen parallel zur Axialrichtung der Halterung, bzw. parallel zur Längserstreckung der Spritze verlaufenden Schwenkachse unter Einwirkung zumindest eines Federelements, vorzugsweise einer Drehfeder, gelagert ist. So kann vorgesehen sein, den Halteflügel beim Einsetzen der Spritze entgegen der Federkraft zu verschwenken, sodass mit Erreichen einer Endmontagestellung der Spritze in der Halterung der Halteflügel mit Hilfe der von der Feder aufgebrachten Haltekraft die Spritze zumindest in Axialrichtung in der vorgegebenen Position hält.

Nach einer bevorzugten Ausgestaltung sind zwei parallel zueinander verlaufende, schwenkbar an der Halterung angelenkte Halteflügel vorgesehen, die je nach Schwenkstellung eine Spritzenaufnahme variabler Größe bilden. Durch das Vorsehen zweier Halteflügel können unterschiedlich große Spritzenkörper in der Halterung problemlos fixiert werden. Es erweist sich dabei ferner von Vorteil, wenn die Halteflügel in der Ebene senkrecht zur Axialrichtung, das heißt senkrecht zur Ebene ihrer Schwenkachse zumindest bereichsweise eine dem Spritzenkörper angepasste Krümmung aufweisen. Die Krümmung der Halteflügel ist von innen, von der Aufnahme her betrachtet, bevorzugt konvex, sodass der in Umfangsrichtung typischerweise rund ausgebildete Spritzenkörper möglichst großflächig an den Halteflügeln zur Anlage gelangt. Je nach bevorzugtem Spritzentyp ist die Krümmung der Halteflügel an die Krümmung des Spritzenkörpers anzupassen oder sie ist hieran angepasst.

Nach einer weiteren bevorzugten Ausgestaltung der Erfindung ist ferner vorgesehen, dass der zumindest eine Halteflügel an einem seiner Schwenkachse entgegengesetzten, also an seinem freien Endabschnitt einen im Wesentlichen radial nach außen ragenden und als Einführhilfe für die Spritze fungierenden Endabschnitt aufweist. In Montagerichtung der Spritze gesehen verjüngt sich der Halteflügel nach innen, bevor er eine konkav gekrümmte Aufnahme für den Spritzenkörper bildet.

Beim Einführen der Spritze kann der zum freien Ende der Halteflügel sich radial erweiternde Endabschnitt nicht nur als Einführhilfe dienen, sondern etwa beim Einsetzen des Spritzenkörpers in die Aufnahme dafür Sorge tragen, dass die Halteflügel nach außen verschwenken und somit erst die Aufnahme für den Spritzenkörper freigeben. Nachdem der Spritzenkörper mit seinem vollen Radius jene Taillierung passiert hat, können die Halteflügel unter Einwirkung der von der Drehfeder bereitgestellten Haltekraft wieder in Richtung ihrer ursprünglichen Konfiguration verschwenken und den Spritzenkörper zwischen sich einklemmen.

Nach einer weiteren vorteilhaften Ausgestaltung der Erfindung ist ferner vorgesehen, zumindest ein Haltemittel in Form eines schwenkbar oder drehbar am Gehäuse gelagerten Verschlussmittels auszubilden, so dass die für die Spritze vorgesehene Aufnahme mittels des Verschlussmittels verschließbar ist. Eine derartige Konfiguration ist insbesondere dann vorgesehen, wenn das Gehäuse der Halterung nur bereichsweise zylindrisch, nach einer Seite hin offen, zum radialen Einsetzen der Spritze ausgebildet ist und sorgt so dafür, dass auch in dieser Ausführungsform die Spritze in der Halterung fixiert ist.

In einer bevorzugten Ausführungsform ist das Verschlussmittel dabei als klappenartiger Verschluss ausgebildet, der an einer Seite der Spritzenaufnahme schwenkbar angelenkt und nach Einsetzen der Spritze in die Aufnahme zur gegenüberliegenden Wange des Gehäuses schwenkbar und dort fixierbar ist.

Als Fixierung ist hierbei vorzugsweise ein Rastmechanismus von Gehäusewange und verschwenkbarem Verschlussmittel vorzusehen. Das Verschlussmittel selbst ist dabei bevorzugt als ein die Spritzenaufnahme in Umfangsrichtung des Gehäuses und in Spritzenlängsrichtung zumindest bereichsweise umschließendes Rastmittel ausgebildet. Mit anderen Worten ist das Verschlussmittel als ein die Aufnahme in Umfangsrichtung des Gehäuses und in Spritzenlängsrichtung zumindest bereichsweise umschließendes Rastmittel ausgebildet, welches mit einem außen am Gehäuse angeordneten Gegenrastmittel zusammenwirkt. Dieses wirkt zum Verschließen der Spritzenaufnahme mit einem außen am Gehäuse bzw. an der entsprechenden Gehäusewange angeordneten Gegenrastmittel zusammen. Wenngleich die Spritze bereits in Radialrichtung gesehen mit Hilfe des zumindest einen Halteflügels an der Halterung fixiert werden kann, so bildet das schwenkbar am Gehäuse angeordnete Verschlussmittel eine weitere Sicherung gegen unbeabsichtigtes Lösen der Spritze aus dem Gehäuse bzw. von der Halterung.

Nach einer weiteren vorteilhaften Ausgestaltung der Erfindung ist ferner vorgesehen, dass das schwenkbar am Gehäuse gelagerte Verschlussmittel an seiner der Spritze zugewandten Innenseite zumindest eine Zahnfläche oder Verzahnung aufweist, welche dazu ausgebildet ist, mit einer korrespondierend hierzu ausgebildeten endseitigen Schrägfläche oder Fase des zumindest einen Halteflügels derart zusammenzuwirken, dass der Halteflügel unter Einwirkung einer radial nach innen gerichteten Kraft auf das Verschlussmittel radial nach innen zur Spritze hin verschwenkt. Mit anderen Worten wirkt das Verschlussmittel innenseitig mit dem radial nach außen gerichteten freien Endabschnitt zumindest eines, bevorzugt zweier Halteflügel zusammen, wobei durch eine nach innen gerichtete Druckausübung auf das Verschlussmittel der Halteflügel radial nach innen verschwenkt und so eine Klemmwirkung zwischen Halteflügel und Spritze herbeigeführt werden kann.

Es erweist sich hierbei als besonders vorteilhaft, wenn das Verschlussmittel zwei in Umfangsrichtung nebeneinander angeordnete und flexibel miteinander verbundene Flächensegmente aufweist, welche jeweils mit einer innenliegenden Zahnfläche mit einer endseitigen Schrägfläche eines Halteflügels zusammenwirken. Die beiden Flächensegmente des Verschlussmittels sind in Umfangsrichtung gesehen nebeneinanderliegend angeordnet und können entlang einer in Axialrichtung verlaufenden Achse gegeneinander verschwenken. Mittels dieser Schwenkbarkeit oder Knickbarkeit des Verschlussmittels kann jedes der nach innen, zumindest bereichsweise verzahnten Flächensegmente eine radial nach innen gerichtete Kraft auf die schwenkbar in der Aufnahme angeordneten Halteflügel ausüben. Auf diese Art und Weise wird ferner ermöglicht, dass ein und dieselbe Halterung zur Fixierung unterschiedlich großer Spritzen fungieren kann.

In einer alternativ bevorzugten Ausführungsform ist das Verschlussmittel als ein um die Axialrichtung im Vergleich zum Gehäuse drehbarer Verschluss ausgebildet, wobei im Verschlussmittel ein Bereich ausgebildet ist, der nach partieller Drehung um die Spritzenlängsrichtung die Spritze in der Aufnahme des Gehäuses fixiert. Mit anderen Worten dient das Verschlussmittel nach partieller Drehung dazu, die Spritze in der Aufnahme des Gehäuses zu fixieren und ein Ver- oder Rausrutschen der Spritze zu verhindern.

Vorteilhafterweise ist das drehbare Verschlussmittel dabei senkrecht zur Axialrichtung der Halterung und um die Längsachse der Spritze drehbar ausgebildet. Besonders bevorzugt ist das drehbare Verschlussmittel einstückig als Schraubverschluss ausgebildet.

Beispielsweise kann der drehbare Verschluss dabei als eine Art offener Ring ausgebildet sein, der die Spritzenaufnahme zumindest teilweise radial und mit einer Öffnung umschließt und der nach Einsetzen der Spritze in die Aufnahme um die Spritzenlängsrichtung drehbar ist, wodurch die Öffnung des Ringes seitlich verdreht und dadurch die Spritze in der Aufnahme fixiert wird. Die Handhabung, insbesondere die einhändige Handhabung, des drehbaren Verschlussmittels erweist sich als besonders einfach und daher besonders vorteilhaft.

Bei einer weiteren bevorzugten Ausgestaltung der Halterung ist ferner vorgesehen, dass das Verschlussmittel den Bereich der Spritzenaufnahme in Axialrichtung gesehen nur bereichsweise überdeckt. Insbesondere soll der unten, dem Standfuß zugewandte Spritzenabschnitt nicht vom Verschlussmittel überdeckt werden, sondern sichtbar bleiben, damit der Anwender den Füllstand der Spritze während des Injektionsvorgangs visuell überprüfen kann. Des Weiteren ist für die Erfindung bevorzugt vorgesehen, dass der zumindest eine Halteflügel und/oder das Gehäuse selbst an seinem oberen, dem Standfuß abgewandten Endabschnitt und/oder das Verschlussmittel, das sich am oberen, dem Standfuß abgewandten Endabschnitt des Gehäuses befindet, zumindest einen Schlitz oder ein Auflager für einen radial nach außen ragenden Haltevorsprung bzw. für zwei diametral in Radialrichtung auskragende Haltevorsprünge einer Spritze aufweist, um die Spritze zumindest in Injektionsrichtung betrachtet, axial an der Halterung zu fixieren.

Für den im oberen Abschnitt der Halteflügel vorgesehenen Schlitz kann ferner eine Einführschräge für die Spritzen-Haltevorsprünge vorgesehen werden, sodass mit Einsetzen der Spritze in die Aufnahme auch im Bereich der Haltevorsprünge und den halteflügelseitigen Schlitzen bereits eine Klemmwirkung erzielt werden kann. Sofern in den Halteflügeln bereits Aufnahmeschlitze für die spritzenseitigen Haltevorsprünge vorgesehen sind, kann die Spritze bidirektional in Axialrichtung an der Halterung fixiert werden.

Bei Ausgestaltungen der Halterung ohne Halteflügel oder ohne Schlitze in den Halteflügeln, kann es aber auch ausreichen, wenn das zumindest bereichsweise umlaufend ausgebildete Gehäuse der Halterung zumindest etwas unterhalb des radial nach außen ragenden Anlageflanschs ein Auflager für die spritzenseitigen Haltevorsprünge bildet. Die Spritze wird somit beim Abstellen der Halterung auf einer Unterlage allein durch ihre Gewichtskraft und bei Ausübung einer auf den Spritzenkolben einwirkenden Injektionskraft jeweils mit ihren Haltevorsprüngen am gehäuseseitigen Auflager in Axialrichtung fixiert, etwa indem sie sich hieran abstützt.

Weitere Ziele, Merkmale sowie vorteilhafte Ausgestaltungen der Erfindung werden in der nachfolgenden Beschreibung von Ausführungsbeispielen unter Bezugnahme auf die Figuren erörtert. Dabei bilden sämtliche im Text beschriebenen als auch in den Figuren bildlich dargestellten Merkmale sowohl in Alleinstellung als auch in jeglicher sinnvollen Kombination untereinander den Gegenstand der vorliegenden Erfindung.

Es zeigen:
- Fig. 1: eine perspektivische Darstellung einer Spritzenhalterung in einer ersten Ausführungsform mit geschlossenem Verschluss,
- Fig. 2: die Spritzenhalterung gemäß Fig. 1 mit geöffnetem Verschluss,
- Fig. 3: die Halterung gemäß Fig. 2 ohne darin eingesetzte Spritze,
- Fig. 4: eine perspektivische Darstellung einer weiteren Ausgestaltung einer Halterung mit in Radialrichtung geschlitzten Halteflügeln,
- Fig. 5: die Halterung gemäß Fig. 4 mit eingesetzter Spritze,
- Fig. 6: eine vergrößerte Detailansicht der Spritzenhalterung gemäß Fig. 5,
- Fig. 7: eine perspektivische Darstellung eines Querschnitts durch die Halterung gemäß der Fig. 4 bis 6 in Höhe der am Verschluss vorgesehenen Verzahnung,
- Fig. 8: eine teiltransparente rückwärtige Ansicht der Halterung gemäß der Fig. 4 bis 7,
- Fig. 9: eine perspektivische Darstellung einer Spritzenhalterung in einer weiteren Ausführungsform mit drehbarem, geöffnetem Verschluss und
- Fig. 10: eine vergrößerte Detailansicht des drehbaren Verschlusses gemäß Fig. 9.

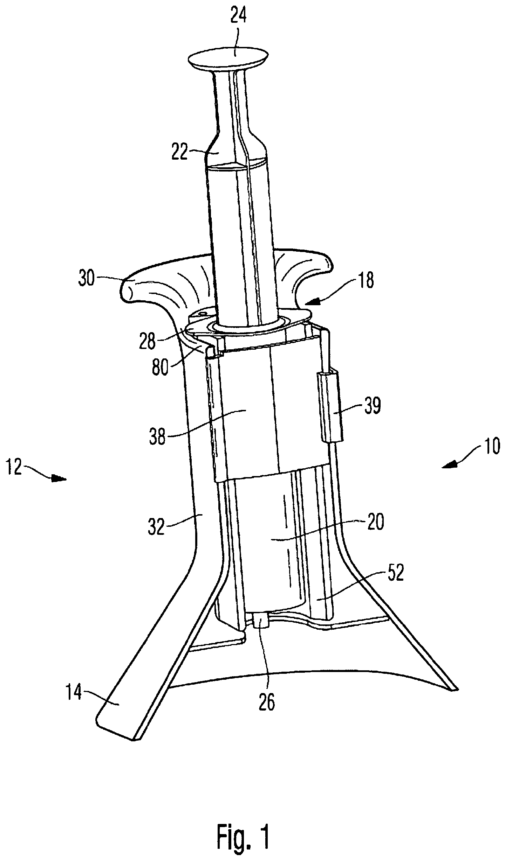

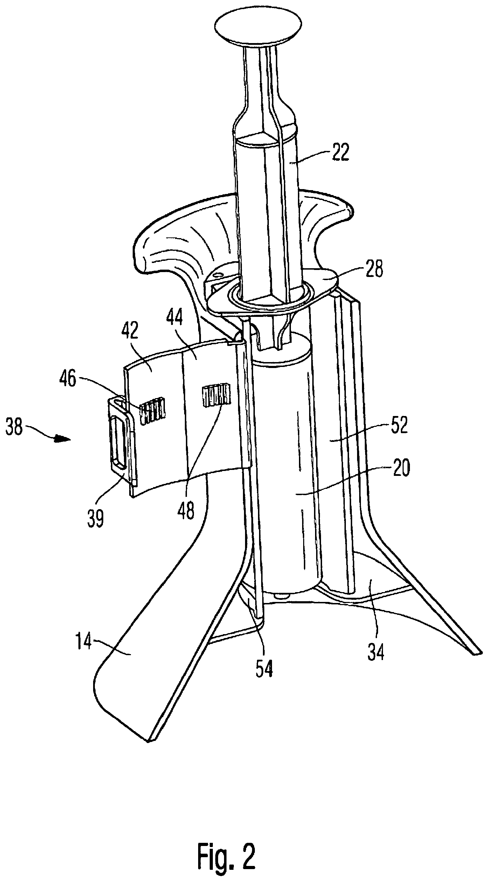

In den Fig. 1 bis 3 ist eine Halterung 10 zur Aufnahme einer Injektionsspritze 18 gezeigt. Die Halterung 10 weist eine Aufnahme 16, etwa für eine Einwegspritze 18 auf, die zum Beispiel einen im Wesentlichen zylindersymmetrischen Spritzenkörper 20 und einen darin axial verschiebbaren Spritzenkolben 22 aufnimmt. An der dem Spritzenauslass 26 des Spritzenkörpers 20 abgewandten Seite weist der Spritzenkolben 22 eine Druck- oder Kraftaufnahmefläche 24 auf, über welche beispielsweise der Anwender mit Hilfe seines Daumens die zum Injizieren einer Flüssigkeit benötigte Kraft auf den Spritzenkolben 22 ausüben kann.

Die Halterung selbst ist als Spritzgussbauteil, vorzugsweise aus thermoplastischem Kunststoff, ausgebildet. Die Halterung 10 weist ein Spritzengehäuse 12 auf, welches nach unten hin einen sich radial verbreiternden, etwa konisch verlaufenden Standfuß 14 und nach oben, dem Spritzenkolben 22 zugewandt, einen radial nach außen ragenden Anlageflansch 30 aufweist. Dazwischen weist das Gehäuse 12 eine Grifffläche 32 auf. Diese kann etwa der Innenkontur einer Handfläche ergonomisch angepasst sein. Zu Injektionszwecken ist von Vorteil vorgesehen, wenn der Anwender mit seinem Daumen von oben eine Kraft auf das Druckstück 24 ausübt und dabei mit der dem Daumen zugewandten Seite des Zeigefingers an der Unterseite des radial auskragenden Anlageflansches 30 abstützend zur Anlage gelangt.

Mittels des sich konisch oder radial erweiternden Abstell- oder Standfußes kann die gesamte Halterung 10 sicher auf einer, in den Figuren nicht explizit gezeigten Unterlage abgestellt werden. Dies erleichtert zum einen den Injektionsvorgang und ermöglicht es dem Anwender, die erforderliche Kraft für eine langsame und gleichmäßige Injektion kontrolliert aufzubringen. Wesentlich hierbei ist es, dass der Spritzenauslass 26 axial beabstandet von der Unterlage, auf welcher die Halterung 10 abzustellen ist, zu liegen kommt. Auf diese Art und Weise kann ein mit einer in das Körpergewebe eindringenden Injektionseinrichtung fluidführend in Verbindung stehendes Schlauchstück sozusagen frei hängend an den Auslass 26 der Spritze 18 angekoppelt werden. Hierbei kommen bevorzugt standardisierte Verbindungskomponenten, wie etwa Luer-Lock-Kupplungen zur Anwendung.

In Axialrichtung, welche in sämtlichen Fig. 1 bis 8 im Wesentlichen in Vertikalrichtung verläuft, stützt sich die in der Aufnahme 16 zu liegen kommende Spritze 18 mit ihren radial vom Spritzenkörper 20 hervorstehenden Haltevorsprüngen 28 an einem oberen Endabschnitt 80 des Gehäuses 12 ab. Hierbei ist dieser als Auflager fungierende Endabschnitt 80 etwas unterhalb des Anlageflanschs 30 angeordnet. Dies ist insoweit von Vorteil, als dass auch Anwender mit verhältnismäßig kurzem Daumen einen Kolben 22 einer vergleichsweise großen Spritze, etwa einer 20 ml-Spritze problemlos zu Injektionszwecken betätigen können.

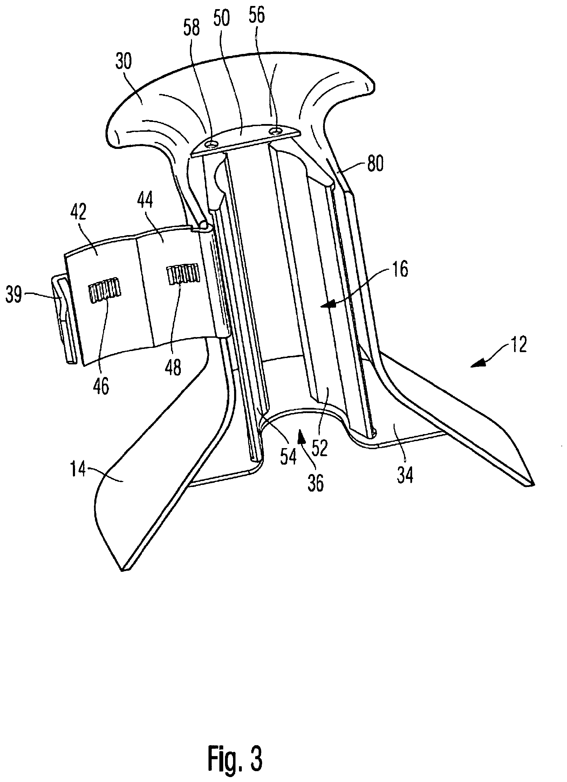

Wie in Fig. 3 ersichtlich, weist das Spritzgussgehäuse 12 im Bereich der konischen Erweiterung des Standfußes 14 eine Stegplatte 34 auf, die einerseits die Verwindungssteifigkeit und Stabilität des Gehäuses 12 erhöht und andererseits als Aufnahme für die Schwenkachsen 56, 58 der beiden Halteflügel 52 und 54 fungieren kann. Die innerhalb der Aufnahme 16 schwenkbar angeordneten Halteflügel 52, 54 sind nach unten an der Stegplatte 34 und nach oben an einem Quersteg 50 angelenkt. Etwaige auf die Halteflügel 52, 54 einwirkende Federelemente 76 sind in den Fig. 1 bis 3 nicht explizit gezeigt. In etwa fluchtend zu der von den Halteflügeln 52, 54 gebildeten Aufnahme 16 ist in der unten liegenden Stegplatte 34 eine Durchgangsöffnung 36 vorgesehen, innerhalb welcher der Spritzenauslass 26 oder je nach Spritzengröße auch der Spritzenkörper 20 zu liegen kommen kann.

In Fig. 3 ist ebenfalls die Innenseite des schwenkbaren Verschlusses 38 gezeigt, welcher an einer Seitenwange des zylindrischen Gehäuseabschnitts um eine Achse 78 schwenkbar angelenkt ist und mit Hilfe eines Rastmittels 39 an der gegenüberliegenden, in Fig. 3 rechts angeordneten Gehäusewange und einem dort vorgesehenen Gegenrastmittel 40 einrasten kann. Der Verschluss 38 ist in zwei Flächensegmente 42, 44 unterteilt, welche jeweils auf ihrer Innenseite eine zumindest sich bereichsweise in Axialrichtung erstreckende und in Umfangsrichtung verlaufende Verzahnung oder Zahnfläche 46, 48 aufweisen.

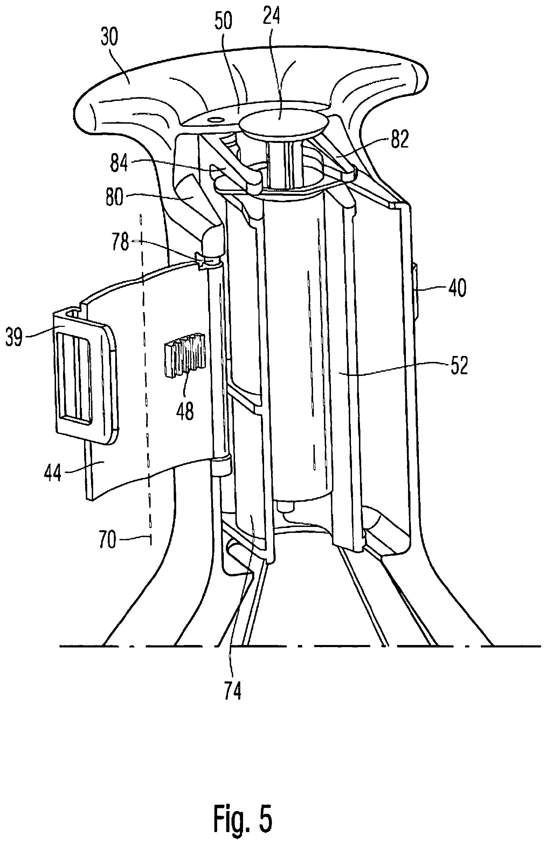

Die beiden Flächensegmente 42, 44 sind dabei biegeelastisch zueinander und schwenkbar ausgebildet. Zwischen den beiden Flächensegmenten verläuft eine, in Fig. 5 gesondert dargestellte Knick- oder Biegeachse 70.

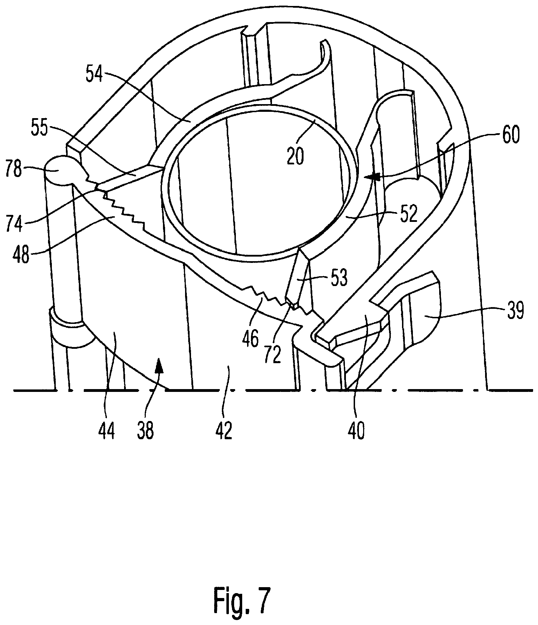

Mit Erreichen seiner in Fig. 7 verdeutlichten Verschlussstellung rastet der Verschluss 38 mit einer Verschlusslasche 39 an einem an der Seitenwange des Gehäuses ausgebildeten Gegenrastelement 40 ein. Es ist hierbei ferner erkennbar, wie die radial nach innen weisenden Zahnflächen 46, 48 mit den korrespondierend hierzu ausgebildeten gefasten Endabschnitten der Halteflügel 52, 54 zusammenwirken. Wird beispielsweise ein radial nach innen gerichteter Druck auf die Flächensegmente 42, 44 des Verschlusses 38 ausgeübt, so bewirkt dies, dass die Halteflügel 52, 54 über die wechselseitige Verzahnung von Zahnfläche 46, 48 und Fase 72, 74 radial nach innen zur Bildung einer Klemmung mit dem Spritzenkörper 20 gedrückt werden. Somit kann die Halterung 10 universell für unterschiedlich große Spritzen und Spritzendurchmesser Verwendung finden.

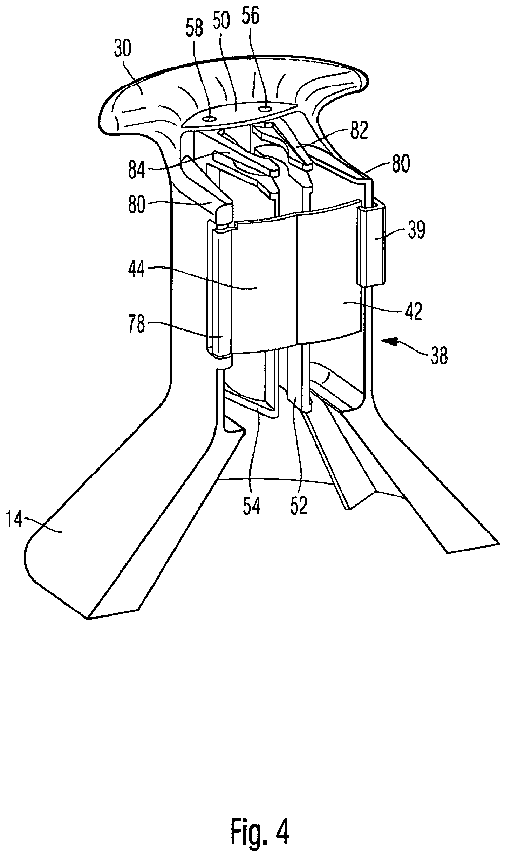

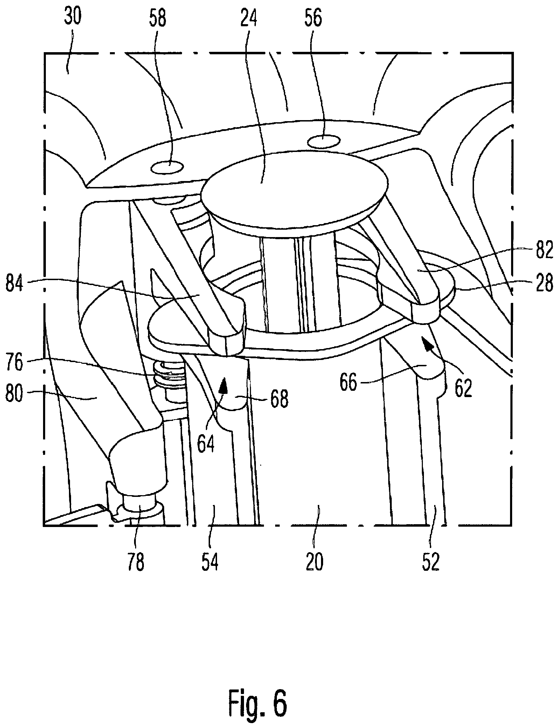

Im Unterschied zur Ausgestaltung gemäß der Fig. 1 bis 3, weist die Ausführungsform nach den Fig. 4 bis 8 im Wesentlichen lediglich andersartig ausgebildete Halteflügel 52, 54 auf, die der Einfachheit halber aber mit gleichen Bezugsziffern versehen sind. Wie in den Fig. 5 und 6 ersichtlich, weisen die Halteflügel 52, 54 in diesem Fall jeweils einen in etwa in Höhe des Auflagers 80 des Gehäuses 12 liegenden Schlitz 62, 64 auf, in welche die diametral gegenüberliegenden Haltevorsprünge 28 des Spritzenkörpers 20 in Radialrichtung eingefügt werden können.

Für ein leichtes Einfügen kann hierbei vorgesehen sein, die Schlitze 62, 64 mit einer Einführschräge 66, 68 zu versehen. Insoweit werden die Halteflügel 52, 54, in Axialrichtung gesehen, zweigeteilt, wobei nunmehr das obere Ende der Halteflügel jeweils von einem fluchtend zum eigentlichen Flügel 52, 54 ausgebildeten Zapfen 82, 84 gebildet wird.

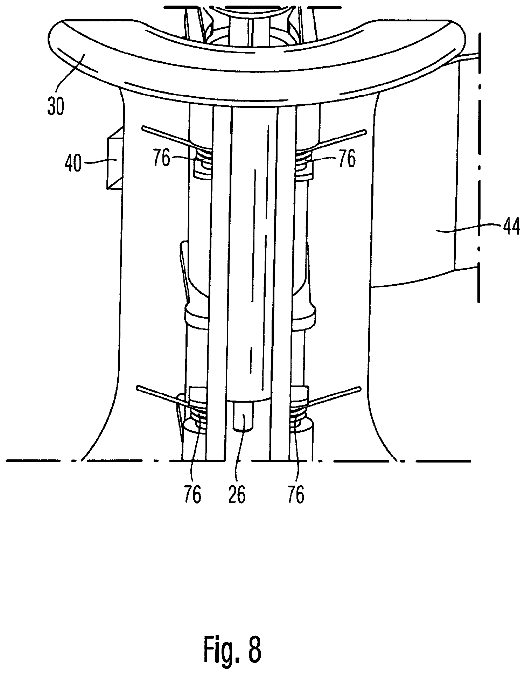

In den Fig. 6 und 8 sind ferner diverse Drehfederelemente 76 gezeigt, mittels derer die Halteflügel 52, 54 unter Federvorspannung stehend am Halter schwenkbar angelenkt sind.

In Fig. 7 ist ferner zu erkennen, dass die Halteflügel 52, 54 bereichsweise konkav ausgebildet sind, um insbesondere mit dem weitgehend zylindrischen Spritzenkörper 20 einen Klemmsitz 60 bilden zu können. Der von den jeweiligen Schwenkachsen 56, 58 beabstandet liegende radiale Endabschnitt der Halteflügel 52, 54 ist dabei vorzugsweise radial nach außen ragend ausgebildet, um die Aufnahme des zylindersymmetrischen Spritzenkörpers 20 zu erleichtern. Die beiden Halteflügel 52, 54 sind beispielsweise vor einem Einsetzen einer Spritze 18 in unmittelbarer Anlagestellung. Die beiden radial nach außen weisenden Enden 53, 55 der jeweiligen Halteflügel 52, 54 bilden eine Art Einführschräge für den Spritzenkörper 20.

Wird die Spritze z.B. in die Aufnahme 16 hineingedrückt, so führt eine solche radial nach innen gerichtete Bewegung des Spritzenkörpers 20 zu einem Aufschwenken der Halteflügel 52, 54. Sobald die Spritze 18 ihre in Fig. 7 gezeigte Endposition erreicht, schwenken die Halteflügel 52, 54 wieder radial nach innen. Durch Verschließen des Verschlusses 38 und der beschriebenen Wechselwirkung der Verzahnungen 46, 48 mit den Endflächen 72, 74 der Halteflügel 52, 54 kann ein ausreichender Klemmsitz 60 zwischen Halteflügeln 52, 54 und Spritzenkörper 20 und ein sichere Fixierung der Spritze in der Halterung 10 bereitgestellt werden.

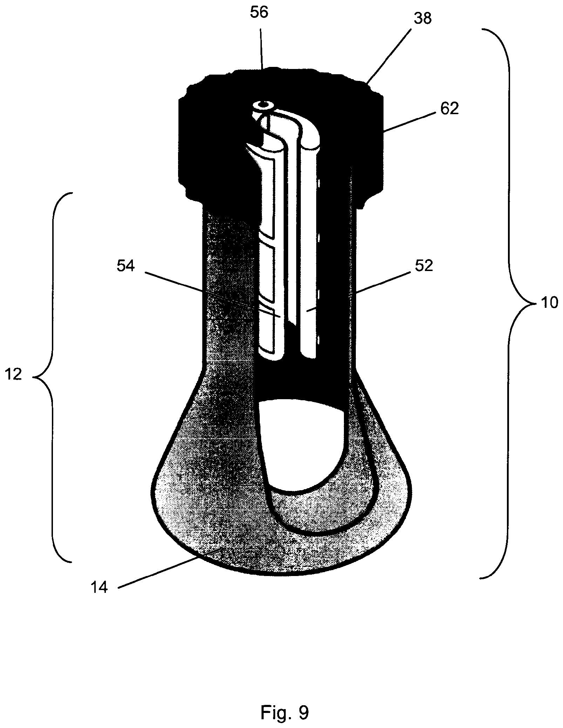

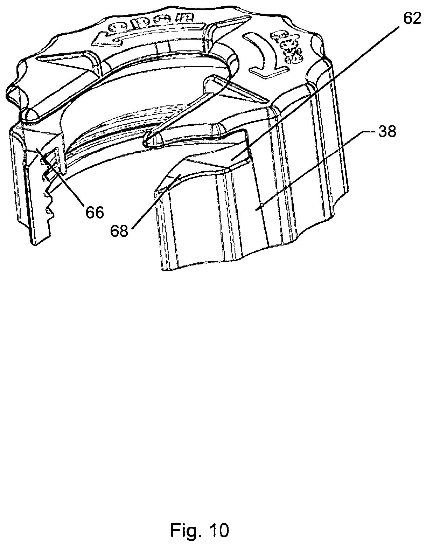

Im Unterschied zur Ausgestaltung gemäß der Fig. 1 bis 3 und Fig. 4 bis 8 weist die Ausführungsform nach den Fig. 9 und 10 im Wesentlichen lediglich ein andersartig ausgebildetes Verschlussmittel 38 in Form eines drehbaren Verschlusses auf, das wie auch die restlichen Elemente der Ausführungsform der Einfachheit halber aber mit der gleichen Bezugsziffer versehen ist.

Konkret zeigt die weitere Ausführungsform der Fig. 9 und 10 auch eine Halterung 10 zur Aufnahme einer Injektionsspritze 18. Die Halterung 10 weist wiederum eine Aufnahme 16, die zum Beispiel einen im Wesentlichen zylindersymmetrischen Spritzenkörper 20 aufnimmt, und ein Gehäuse 12 auf, welches nach unten hin einen sich radial verbreiternden, etwa konisch verlaufenden Standfuß 14 aufweist. In Axialrichtung, welche in den Fig. 9 und 10 im Wesentlichen in Vertikalrichtung verläuft, stützt sich die in der Aufnahme 16 zu liegen kommende Spritze 18 mit ihren radial vom Spritzenkörper 20 hervorstehenden Haltevorsprüngen 28 entweder auf den oberen Endabschnitten der Halteflügel 52, 54 (bei kleineren Spritzen) und/oder in dem Schlitz 62 des Verschlusses 38 (bei größeren Spritzen) ab.

Die innerhalb der Aufnahme 16 angeordneten beiden Halteflügel 52, 54 weisen in der hier gezeigten Ausführungsform eine gemeinsame Schwenkachse 56 auf. Die auf die Halteflügel 52, 54 einwirkenden Federelemente 76 sind genauso wie die Querstege 50 zur Aufnahme der Schwenkachse 56 nicht explizit in Fig. 9 gezeigt. Gut zu erkennen ist aber, dass die Halteflügel 52, 54 wiederum bereichsweise konkav ausgebildet sind, um insbesondere mit dem weitgehend zylindrischen Spritzenkörper 20 einen Klemmsitz 60 bilden zu können. Auch hier ist der von der Schwenkachse 56 beabstandet liegende radiale Endabschnitt der Halteflügel 52, 54 radial nach außen ragend ausgebildet, um die Aufnahme des Spritzenkörpers 20 zu erleichtern.

In Fig. 10 ist der drehbare Verschluss 38 im Detail genauer gezeigt. Der Verschluss weist in diesem Fall ein Schraubgewinde zur Befestigung des Verschlusses 38 am Gehäuse 12 und einen etwa in Höhe der oberen Endabschnitte der Halteflügel 52, 54 liegenden und den ganzen Verschluss 38 umlaufenden Schlitz 62 auf, in welche die diametral gegenüberliegenden Haltevorsprünge 28 des Spritzenkörpers 20 in Radialrichtung eingefügt werden können. Für ein leichtes Einfügen kann hierbei vorgesehen sein, den Schlitz 62 mit zwei Einführschrägen 66, 68 zu versehen.

Wird die Spritze z.B. in die Aufnahme 16 dieser Ausführungsform hineingedrückt, so führt auch hier eine solche radial nach innen gerichtete Bewegung des Spritzenkörpers 20 zu einem Aufschwenken der Halteflügel 52, 54. Sobald die Spritze 18 ihre Endposition erreicht, schwenken die Halteflügel 52, 54 wieder radial nach innen. Hierdurch und durch partielle Drehung des Verschlusses 38 kann ein ausreichender Klemmsitz 60 zwischen Halteflügeln 52, 54 und Spritzenkörper 20 und ein sichere Fixierung der Spritze in der Halterung 10 in axialer und radialer Richtung dieser Ausführungsform bereitgestellt werden.

### Bezugszeichenliste

- 10: Halterung
- 12: Gehäuse
- 14: Standfuß
- 16: Aufnahme
- 18: Spritze
- 20: Spritzenkörper
- 22: Spritzenkolben
- 24: Druckstück
- 26: Auslass
- 28: Haltevorsprung
- 30: Anlageflansch
- 32: Grifffläche
- 34: Steg
- 36: Durchgangsöffnung
- 38: Verschluss
- 39: Rastmittel
- 40: Gegenrastmittel
- 42: Verschlusssegment
- 44: Verschlusssegment
- 46: Zahnfläche
- 48: Zahnfläche
- 50: Quersteg
- 52: Halteflügel
- 53: Endabschnitt
- 54: Halteflügel
- 55: Endabschnitt
- 56: Schwenkachse
- 58: Schwenkachse
- 60: Klemmsitz
- 62: Schlitz
- 64: Schlitz
- 66: Einführschräge
- 68: Einführschräge
- 70: Biegeachse
- 72: Schrägfläche
- 74: Schrägfläche
- 76: Drehfeder
- 78: Schwenkachse
- 80: Auflager
- 82: Zapfen
- 84: Zapfen

## Patentansprüche

1. Halterung für eine Injektionsspritze (18) zum Ausüben einer nach unten gerichteten Injektionskraft mit dem Daumen oder einer anderen Körperpartie, etwa mit der flachen Hand, zu Selbstmedikationszwecken mit einem zur Aufnahme der Spritze ausgebildeten Gehäuse (12), einem Standfuß (14) zum Abstellen auf einer Unterlage und mit Haltemitteln (38, 52, 54, 62, 64) zum axialen und/oder radialen Fixieren der Spritze (18) an oder in der Halterung, wobei die Haltemittel (38, 52, 54, 62, 64) derart ausgebildet sind, dass ein Auslass (26) einer in der Halterung vertikal angeordneten Spritze (18) dem freien Ende des Standfußes (14) zugewandt und beabstandet hiervon zu liegen kommt
, wobei der Abstand des Auslasses (26) zum Standfußende wenigstens der Axialerstreckung eines Schlauch-Anschlussstücks zuzüglich eines Mindest-Schlauchbiegeradius entspricht, bei welchem der mit dem Spritzenauslass in Fluidverbindung tretende Schlauch fluiddurchlässig bleibt
, wobei das Gehäuse (12) an seinem dem Standfuß (14) abgewandten oberen Endabschnitt einen radial nach außen ragenden, zumindest bereichsweise umlaufenden Anlageflansch (30) aufweist.

2. Halterung nach einem der vorhergehenden Ansprüche, wobei das Gehäuse (12) eine zumindest bereichsweise zylindrische, mit einer außenseitigen Grifffläche (32) versehene Grundstruktur aufweist, die sich zum Standfuß (14) hin radial erweitert.

3. Halterung nach einem der vorhergehenden Ansprüche, wobei das Gehäuse (12) eine radial zugängliche Aufnahme (16) zum Einfügen der Spritze (18) in das Gehäuse (12) aufweist.

4. Halterung nach einem der vorhergehenden Ansprüche, wobei in der Aufnahme (16) zumindest ein Halteflügel (52, 54) schwenkbar gelagert ist, mit welchem die Spritze (18) klemmend in die Halterung bringbar ist.

5. Halterung nach Anspruch 4, wobei der zumindest eine Halteflügel (52, 54) an einer im Wesentlichen parallel zur Axialrichtung verlaufenden Schwenkachse (56, 58) unter Einwirkung zumindest einer Drehfeder (76) gelagert ist.

6. Halterung nach einem der vorherigen Ansprüche 4 oder 5, wobei zwei parallel zueinander verlaufende Halteflügel (52, 54) derart ausgebildet sind, dass die Aufnahme (16) das Einfügen einer Spritze variabler Größe ermöglicht.

7. Halterung nach einem der vorhergehenden Ansprüche 4 bis 6, wobei der zumindest eine Halteflügel (52, 54) in der Ebene senkrecht zur Axialrichtung zumindest bereichsweise eine dem Spritzenkörper (20) angepasste Krümmung aufweist.

8. Halterung nach einem der vorhergehenden Ansprüche 4 bis 7, wobei der zumindest eine Halteflügel (52, 54) an einem seiner Schwenkachse (56, 58) entgegengesetzten Endabschnitt einen im Wesentlichen radial nach außen ragenden und als Einführhilfe für die Spritze (18) fungierenden Endabschnitt (53, 55) aufweist.

9. Halterung nach einem der vorhergehenden Ansprüche 3 bis 8, wobei die für die Spritze (18) vorgesehene Aufnahme (16) mittels eines schwenkbar oder drehbar am Gehäuse (12) gelagerten Verschlussmittels (38) verschließbar ist.

10. Halterung nach Anspruch 9, wobei das Verschlussmittel (38) drehbar um die Axialrichtung im Vergleich zum Gehäuse (12) ausgebildet ist und wobei im Verschlussmittel (38) einen Bereich ausgebildet ist, der nach partieller Drehung um die Spritzenlängsrichtung die Spritze (18) in der Aufnahme (16) des Gehäuses (12) fixiert.

11. Halterung nach Anspruch 9 oder 10, wobei das drehbare Verschlussmittel (38) senkrecht zur Axialrichtung der Halterung und um die Längsachse der Spritze drehbar ausgebildet ist.

12. Halterung nach einem der vorhergehenden Ansprüche 9 bis 11, wobei das drehbare Verschlussmittel (38) einstückig als Schraubverschluss ausgebildet ist.

13. Halterung nach einem der vorhergehenden Ansprüche, wobei der zumindest eine Halteflügel (52, 54) und/oder das Gehäuse selbst (12) und/oder das Verschlussmittel (38) an ihren oberen, dem Standfuß (14) abgewandten Endabschnitten zumindest einen Schlitz (62, 64) oder ein Auflager (80) für einen radial nach außen ragenden Haltevorsprung (28) einer Spritze (18) aufweisen, um die Spritze (18) zumindest in ihrer Injektionsrichtung axial an der Halterung zu fixieren.

## Claims

1. Holder for an injection syringe (18) for applying a downwardly directed injection force with the thumb or another part of the body, for example the flat of a hand, for purposes of self-medication, with a housing (12) designed to receive the syringe, with a stand (14) for placing on a supporting surface, and with retaining means (38, 52, 54, 62, 64) for axially and/or radially fixing the syringe (18) on or in the holder, wherein the retaining means (38, 52, 54, 62, 64) are designed in such a way that an outlet (26) of a syringe (18) arranged vertically in the holder comes to lie facing the free end of the stand (14) and at a distance therefrom,
wherein the distance of the outlet (26) from the end of the stand corresponds at least to the axial extent of a hose attachment piece plus a minimum hose bend radius at which the hose in fluid connection with the syringe outlet remains open to fluid,
wherein the housing (12), at its upper end portion directed away from the stand (14), has a radially outwardly protruding contact flange (30), which is arranged at least in part about the periphery.

2. Holder according to one of the preceding claims, wherein the housing (12) has an at least partially cylindrical main structure, which is provided with a grip surface (32) on the outside and which radially widens towards the stand (14).

3. Holder according to either of the preceding claims, wherein the housing (12) has a radially accessible seat (16) for insertion of the syringe (18) into the housing (12).

4. Holder according to one of the preceding claims, wherein at least one retaining wing (52, 54) is mounted pivotably in the seat (16), with which at least one retaining wing (52, 54) the syringe (18) can be clamped into the holder.

5. Holder according to Claim 4, wherein the at least one retaining wing (52, 54) is mounted on a pivot axle (56, 58), extending substantially parallel to the axial direction, under the effect of at least one torsion spring (76).

6. Holder according to either of Claims 4 and 5, wherein two retaining wings (52, 54) extending parallel to each other are designed in such a way that the seat (16) permits the insertion of a syringe of variable size.

7. Holder according to one of Claims 4 to 6, wherein the at least one retaining wing (52, 54) has at least in part, in the plane perpendicular to the axial direction, a curvature adapted to the syringe body (20).

8. Holder according to one of Claims 4 to 7, wherein the at least one retaining wing (52, 54), at an end portion opposite its pivot axle (56, 58), has a substantially radially outwardly protruding end portion (53, 55), which functions as an insertion aid for the syringe (18).

9. Holder according to one of Claims 3 to 8, wherein the seat (16) provided for the syringe (18) can be closed by means of a closure piece (38) mounted pivotably or rotatably on the housing (12).

10. Holder according to Claim 9, wherein the closure piece (38) is designed to be rotatable about the axial direction in relation to the housing (12), and wherein the closure piece (38) is designed with a region which, after partial rotation about the longitudinal direction of the syringe, fixes the syringe (18) in the seat (16) of the housing (12).

11. Holder according to Claim 9 or 10, wherein the rotatable closure piece (38) is designed such that it can rotate perpendicularly with respect to the axial direction of the holder and about the longitudinal axis of the syringe.

12. Holder according to one of Claims 9 to 11, wherein the rotatable closure piece (38) is designed in one piece as a screw cap.

13. Holder according to one of the preceding claims, wherein the at least one retaining wing (52, 54) and/or the housing (12) itself and/or the closure piece (38) have/has, at the upper end portions thereof directed away from the stand (14), at least one slit (62, 64) or a bearing (80) for a radially outwardly protruding retaining projection (28) of a syringe (18), in order to fix the syringe (18) axially on the holder, at least in its injection direction.

## Revendications

1. Support pour une seringue d'injection (18) pour exercer une force d'injection orientée vers le bas avec le pouce ou avec une autre partie du corps, par exemple avec le plat de la main, à des fins d'automédication, comprenant un boîtier (12) réalisé pour recevoir la seringue, un pied d'appui (14) prévu pour être posé sur un subjectile, et des moyens de retenue (38, 52, 54, 62, 64) pour la fixation axiale et/ou radiale de la seringue (18) au ou dans le support, les moyens de retenue (38, 52, 54, 62, 64) étant réalisés de telle sorte qu'une sortie (26) d'une seringue (18) disposée verticalement dans le support soit tournée vers l'extrémité libre du pied d'appui (14) et vienne se placer à distance de celle-ci, la distance de la sortie (26) par rapport à l'extrémité du pied d'appui correspondant au moins à l'étendue axiale d'une pièce de raccordement de tuyau plus un rayon de courbure de tuyau minimal auquel le tuyau entrant en liaison fluidique avec la sortie de seringue reste perméable aux fluides, le boîtier (12) présentant au niveau de sa portion d'extrémité supérieure opposée au pied d'appui (14), une bride d'appui (30) au moins en partie périphérique, saillant radialement vers l'extérieur.

2. Support selon l'une quelconque des revendications précédentes, dans lequel le boîtier (12) présente une structure de base au moins en partie cylindrique, pourvue d'une surface de préhension (32) du côté extérieur, qui s'élargit radialement vers le pied d'appui (14).

3. Support selon l'une quelconque des revendications précédentes, dans lequel le boîtier (12) présente un logement radialement accessible (16) pour l'insertion de la seringue (18) dans le boîtier (12).

4. Support selon l'une quelconque des revendications précédentes, dans lequel au moins une aile de retenue (52, 54) est supportée de manière pivotante dans le logement (16), avec laquelle la seringue (18) peut être montée avec serrage dans le support.

5. Support selon la revendication 4, dans lequel l'au moins une aile de retenue (52, 54) est supportée au niveau d'un axe de pivotement (56, 58) s'étendant essentiellement parallèlement à la direction axiale sous l'action d'au moins un ressort de torsion (76).

6. Support selon l'une quelconque des revendications précédentes 4 et 5, dans lequel deux ailes de retenue s'étendant parallèlement l'une à l'autre (52, 54) sont réalisées de telle sorte que le logement (16) permette l'insertion d'une seringue de taille variable.

7. Support selon l'une quelconque des revendications précédentes 4 à 6, dans lequel l'au moins une aile de retenue (52, 54) présente dans le plan perpendiculaire à la direction axiale, au moins en partie, une courbure adaptée au corps de seringue (20).

8. Support selon l'une quelconque des revendications précédentes 4 à 7, dans lequel l'au moins une aile de retenue (52, 54) présente, au niveau d'une portion d'extrémité opposée à son axe de pivotement (56, 58), une portion d'extrémité (53, 55) faisant saillie essentiellement radialement vers l'extérieur et servant d'auxiliaire d'introduction pour la seringue (18).

9. Support selon l'une quelconque des revendications précédentes 3 à 8, dans lequel le logement (16) prévu pour la seringue (18) peut être fermé au moyen d'un moyen de fermeture (38) supporté de manière pivotante ou rotative sur le boîtier (12).

10. Support selon la revendication 9, dans lequel le moyen de fermeture (38) est réalisé de manière à pouvoir tourner autour de la direction axiale par rapport au boîtier (12) et dans lequel une région est réalisée dans le moyen de fermeture (38), laquelle, après rotation partielle autour de la direction longitudinale de la seringue, fixe la seringue (18) dans le logement (16) du boîtier (12).

11. Support selon la revendication 9 ou 10, dans lequel le moyen de fermeture rotatif (38) est réalisé perpendiculairement à la direction axiale du support et de manière à pouvoir tourner autour de l'axe longitudinal de la seringue.

12. Support selon l'une quelconque des revendications précédentes 9 à 11, dans lequel le moyen de fermeture rotatif (38) est réalisé d'une seule pièce en tant que fermeture vissable.

13. Support selon l'une quelconque des revendications précédentes, dans lequel l'au moins une aile de retenue (52, 54) et/ou le boîtier lui-même (12) et/ou le moyen de fermeture (38) présentent, au niveau de leurs portions d'extrémité supérieures opposées au pied d'appui (14), au moins une fente (62, 64) ou un appui (80) pour une saillie de retenue (28) d'une seringue (18), saillant radialement vers l'extérieur, afin de fixer la seringue (18), au moins dans sa direction d'injection, axialement sur le support.
